# EUROPEAN PATENT APPLICATION

(11) **EP 4 763 211 A1**
(43) Date of publication of application: **24.06.2026**
(21) Application number: 24383434.8
(22) Date of filing: 20.12.2024
(51) Int. Cl.: A61K 35/22, A61K 35/34, A61K 35/407, A61K 35/42, A61P 43/00, C12N 5/00

(54) **BIOFABRICATION OF A FUNCTIONAL VASCULAR TREE MODEL AND USES THEREOF**

(71) Applicant: Fundación Instituto de Investigación Sanitaria Aragón, 50009 Zaragoza Zaragoza (ES)
(72) Inventor: BAPTISTA, Pedro Miguel, 50009 Zaragoza (ES); MELITÓN BARBANCHO, Sandra, 50009 Zaragoza (ES); PLA PALACÍN, Iris, 50009 Zaragoza (ES); LANAS ARBELOA, Ángel, 50009 Zaragoza (ES)
(74) Representative: Hoffmann Eitle

(57) **Abstract**

The present invention refers to an *ex vivo* method to recellularize a perfusion decellularized extracellular matrix of an organ or of a portion of an organ, comprising providing said decellularized extracellular matrix of an organ or of a portion of an organ, and contacting said decellularized extracellular matrix of an organ or of a portion of an organ with a population of cells, preferably regenerative/progenitor cells, under conditions in which said population of cells, preferably regenerative/progenitor cells, engraft, multiply and/or differentiate within and on said decellularized extracellular matrix portion, wherein said conditions comprises:
a. injecting or perfusing the cells into the decellularized extracellular matrix at a pressure of between 1 and 200 mmHg so as to engraft the cells in the said decellularized extracellular matrix;
b. expand the cells by using a proliferation media; and
c. differentiate the cells by using a differentiation media.

## Description

### Technical field of the invention

The present invention pertains to the field of tissue and organ engineering. More specifically, it relates to a functional vascular tree, method to produce it ex-vivo and medical thereof.

### Background of the invention

Tissue engineering emerged in the late 20th century as an interdisciplinary field combining principles of biology, engineering, and materials science. Its goal is to develop biological substitutes that can restore, maintain, or improve the function of damaged tissues or organs. Early efforts focused on creating simple tissue constructs like skin grafts or cartilage replacements, using scaffolds seeded with cells to regenerate or repair tissues. As technology advanced, so did the complexity of tissues that could be engineered, leading to the development of more sophisticated biomaterials and methods, such as organ decellularization and 3D bioprinting.

Nowadays, tissue engineering plays a vital role in regenerative medicine, drug testing, and personalized medical treatments. It holds the promise of overcoming the limitations of organ transplantation, such as donor shortages and immune rejection. Researchers are working on creating functional replacements for critical tissues like bone, cartilage, heart tissue, and even entire organs. The ability to engineer tissues in the lab also supports disease modeling and drug development, allowing more accurate testing and reducing the need for animal models.

One of the most significant challenges in tissue engineering today is the creation of functional vascular trees-the intricate networks of blood vessels necessary to supply oxygen and nutrients to large tissue constructs. Without vascularization, engineered tissues larger than a few millimeters cannot survive due to inadequate nutrient delivery and waste removal.

Creating these vascular networks is critical for advancing the engineering of complex tissues like the heart, liver, and kidneys, where extensive vasculature is essential for functionality. Researchers are developing advanced bioprinting techniques and scaffold designs to create branching vascular networks that closely mimic natural blood vessels.

The complexity of replicating the hierarchical structure of natural blood vessels in-vitro, from large arteries to tiny capillaries, is challenging, especially at the microscale. Engineered vessels may collapse, leak, or fail to support proper blood flow, affecting their stability and long-term functionality. Furthermore, the creation of a vascular tree mimicking the physiological cellular composition and function of a native one is a complex task.

The inefficient generation, or the insufficient maturity/function of the vascular tree will promote blood coagulation, resulting in thrombus formation once transplanted in vivo, blocking the delivery of nutrients and oxygen to the tissue or organ, ultimately causing necrosis. Scaling-up vascular networks for large tissues, such as whole organs, is much more challenging due to the complex vascular trees found in these organs, which are composed of vessels with varying diameters, cellular compositions, and functions. To date, no studies have successfully maintained vessels consisting of more than two different cell types that can sustain blood flow in vivo for more than two weeks without the direct external addition of anticoagulants like heparin after transplantation.

Given the critical role of generating a functional vascular tree in tissue engineering, there is a pressing need for the development of a vascular tree engineering method that can overcome the above-mentioned shortcomings.

### Summary of the invention

In a first aspect, the present invention relates to an ex vivo method of re-cellularizing a perfusion decellularized extracellular matrix of an organ or of a portion of an organ, comprising providing said decellularized extracellular matrix of an organ or of a portion of an organ, and contacting said decellularized extracellular matrix of an organ or of a portion of an organ with a population of cells, preferably regenerative cells, under conditions in which said population of cells, preferably regenerative cells, engraft, multiply and/or differentiate within and on said decellularized extracellular matrix portion, wherein said conditions comprises:
a) injecting or perfusing the cells into the decellularized extracellular matrix at a pressure of between 1 and 200 mmHg so as to engraft the cells in the said decellularized extracellular matrix;
b) expand the cells by using a proliferation media; and
c) differentiate the cells by using a differentiation media.

Preferably said decellularized extracellular matrix of an organ or of a portion of an organ is re-cellularized with at least two different types of regenerative cells.

Preferably, step a) of the method comprises cannulating said organ at one or more cavities, vessels, and/or ducts, thereby producing a cannulated portion; and perfusing said cannulated portion with a seeding medium comprising the regenerative cells so as to provide a re-cellularized organ or of a portion of an organ.

Preferably, the re-cellularized extracellular organ or of a portion of an organ derived from step c) is preconditioned, at physiological pressures, preferably for a duration of at least 1 to 14 days, prior to *in-vivo* transplant by using an adequate media.

Preferably, said decellularized extracellular matrix of an organ or of a portion of an organ is the liver, preferably the liver of a pig, bovine, sheep, canine or human being. Preferably, the method comprises cannulating said organ at the suprahepatic vena cava (SHVC), the hepatic artery (HA) and/or portal vein (PV).

Preferably, the cells are endothelial cells selected from the list consisting of Umbilical Vein Endothelial Cells (UVECs), Outgrowth Endothelial Cells (OECs) and stromal cells selected from the list consisting of Mesenchymal Stem Cells (MSCs) and Smooth Muscle Cells (SMCs).

Preferably, the cells are endothelial cells selected from the list consisting of Umbilical Vein Endothelial Cells (UVECs), Outgrowth Endothelial Cells (OECs) and stromal cells selected from the list consisting of Mesenchymal Stem Cells (MSCs) and Smooth Muscle Cells (SMCs), and in step a) each cell type is divided for each of the following injection routes PV, SHVC and HA and injected or perfused in each of those injection routes at a concentration of between 1x10⁶ to 1x10⁸ during a time interval of 1 to 3 days. Preferably, said administration is carried out by injecting per injection route, in one or more injections per day, the total or a portion of the total amount of cells during a time interval of 1 to 3 days, at decreasing pressures within the range of between 150 and 10 mmHg. Preferably, the cells are administered during a time interval of three days, wherein in day 1 about 33% of the total amount of UVEC cells, 33% of the total amount of OECs cells, 50% of the total amount of MSC cells and 30% of the total amount of SMC cells are administered at a pressure of about 100 mmHg, wherein in day 2 about 33% of the total amount of UVEC cells, 33% of the total amount of OECs cells, 30% of the total amount of MSC cells and 40% of the total amount of SMC cells are administered at a pressure of about 50 mmHg, and wherein in day 3 the rest of the cells of the total amount of cells are administered at a pressure of about 25 mmHg.

Preferably, each day the amount of cells are administered in at least 2-3 injections, and wherein each day one or more extra recirculation injections at the seeding pressure with basal medium are carried out to ensure proper cell penetration.

Preferably, the cells are endothelial cells selected from the list consisting of Umbilical Vein Endothelial Cells (UVECs), Outgrowth Endothelial Cells (OECs) and stromal cells selected from the list consisting of Mesenchymal Stem Cells (MSCs) and Smooth Muscle Cells (SMCs), and the total amount administered per each cell type is divided for each of the following injection routes PV, SHVC/IHVC and HA in the following proportions, about 30% of the total amount of UVEC and OECS are administered via PV, about 50% of the total amount of endothelial cells are administered via SHVC/IHVC, about 20% of the total amount of endothelial cells are administered via HA, about 40% of the total amount of MSC are administered via PV, about 40% of the total amount of stromal cells are administered via SHVC/IHVC, and about 20% of the total amount of stromal cells are administered via HA. Also, about 30% of the total amount of SMC are administered via PV, about 30% of the total amount of stromal cells is administered via SHVC, and about 40% of the total amount of stromal cells are administered via HA.

In a **second aspect,** the present invention provides a functional vascular tree within a re-cellularized extracellular matrix of an organ or of a portion of an organ obtained or obtainable from the method according to the first aspect or any of its embodiments. Preferably, the re-cellularized extracellular matrix is from a liver organ.

In a **third aspect,** the present invention provides the use in organ transplantation in a subject in need thereof.

### Brief description of the drawings

To enable a better understanding of the present disclosure, and to show how the present disclosure may be carried out, reference will now be made, by way of example only, to the accompanying schematic drawings, wherein:
**Figure 1** shows the results of evaluation of different seeding pressures (14/7, 25/14, 50/25, 100/50 mmHg) and cell distribution within the vascular tree. Also result from DAPI staining and LDH tests are shown, proving that the higher-pressures combination condition (50/25 and 100/50) used for cell delivery produced the highest cell engraftment within the scaffold.
**Figure 2** shows a table with the seeding cells schedule, with the percentage and number of cells injected per day in each route (SHVC, PV, HA), as well as the seeding pressure.
**Figure 3** shows the results obtained after quantifying the LDH concentration in the medium at different days in both models, being model 1 the low-pressure regimen and model 2 the high-pressure regimen.
**Figure 4** shows the histological results (a and b) from the low-pressure maintenance regime protocol.
**Figure 5** shows the histological results (a and b) from the high-pressure maintenance regime protocol.
**Figure 6** shows a bar chart with the mRNA expression levels of different genes involved in angiogenesis analyzed by semi-quantitative RT-PCR. The expression level of the target gene was normalized to the housekeeping gene's (GAPDH) expression level.
**Figure 7** shows a seeding scheme showing the different media compositions with growth factors that were used throughout the different experiments aimed at proliferation and/or maturation.
**Figure 8** shows a scheme of the process of exploring porcine OECs (pOECs) potential for revascularization. The obtained colonies were expanded and characterized by flow cytometry and immunofluorescence. The in vitro model images show their proliferative capacities (both in Matrigel and decellularized extracellular matrix (dECM) were superior to conventional porcine UVECs (pUVECs) cell cultures.
**Figure 9** shows the histological results of comparing pUVECs and pOECs of a revascularized rat liver.
**Figure 10** shows a readjusted table with the seeding cells schedule used in rat scaffolds, with the percentage and number of cells injected per day, as well as the seeding pressure, after the introduction of pOECs.
**Figure 11** shows the cell injection percentages per route and pressure for pUVECs, pOECs, pBM-MSCs and pA-SMCs after up-scaling for large organ recellularization.
**Figure 12** shows the average pressure recorded for each day for the HA and PV models, during the preconditioning at physiological pressures.
**Figure 13** shows the process of decellularization and further heparinization of the liver. Furthermore, the histological images of native liver, decellularized and heparinized dECM are shown.

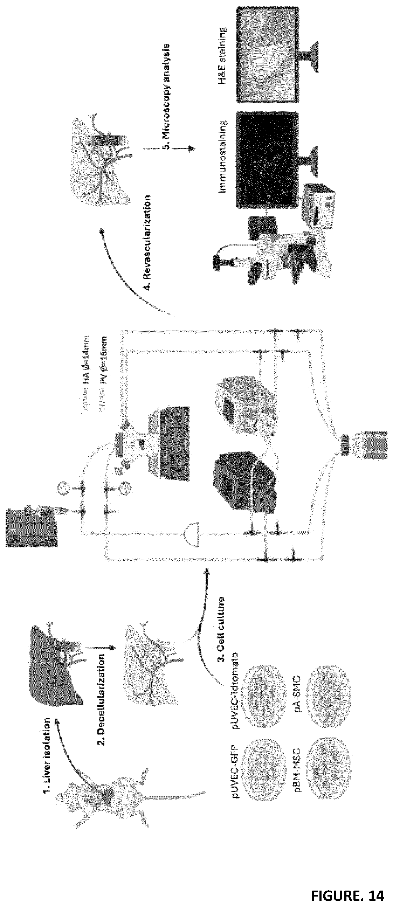

**Figure 14** shows a scheme of the whole process followed for de-cellularization, re-cellularization, perfusion, and microscopy analysis of the liver of a rat.
**Figure 15** shows a scheme of the whole process followed for de-cellularization, re-cellularization, perfusion and further transplant, and microscope analysis of a liver of a pig.

### Description of the invention

### Definitions

It must be noted that, as used herein, the singular forms "a", "an", and "the", include plural references unless the context clearly indicates otherwise. Further, unless stated otherwise, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", if used herein, refers to +/- 30%, preferably +/- 20%, preferably +/- 15%, more preferably +/- 10%, of the indicated referred value. It may also refer to the exact value, that is, +/- 0% or the standard measurement error. Also, any quantity, composition percentages and/or dimension mentioned herein is understood to present an error of +/- 30%, preferably +/- 20%, preferably +/- 15%, more preferably +/- 10%, more preferably +/- 5% of the indicated value. It may also refer to the exact value, that is, +/- 0% or the standard measurement error. For example, a 50% w/v may correspond to an interval of 50% +/- 30%, that is, to the interval 35% to 65% w/v, preferably corresponds to an interval of 50% +/- 20%, that is, to the interval 40% to 60%, more preferably corresponds to an interval of 50 % +/- 15%, that is, to the interval 42.5% to 57.5% w/v, even more preferably corresponds to an interval of 50% +/- 10%, that is, to the interval 45% to 55% w/v, even more preferably corresponds to an interval of 50% +/- 5%, that is, to the interval 47.5% to 52.5% w/v.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of" does not exclude materials or steps that do not materially affect the basic and novel characteristics of the claim.

The term "vascular tree", in the context of the invention, refers to the intricate network of blood vessels that supply and drain blood throughout an organ or portion of an organ, ensuring proper circulation for the organ's metabolic, detoxification, and synthetic functions. This vascular tree is preferably made by at least two different types of cells.

The term "suprahepatic vena cava (SHVC)", in the context of the invention, refers to the vena cava found on the top of the liver, and the term "infrahepatic vena cava (IHVC)" refers to the vena cava found on the inferior side of the liver. They are part of the liver's vascular system and play a crucial role in draining the deoxygenated blood from the liver tissue. They are also vital to access the central veins at the center of each hepatic lobular unit.

The term "hepatic artery (HA)", in the context of the invention, refers to the artery that supplies oxygen-rich blood to the liver.

The term "portal vein (PV)", in the context of the invention, refers to a large vein that carries nutrient-rich, deoxygenated blood to the liver from the mesenteric veins.

The term "ex-vivo method", in the context of the present invention, refers to a technique or procedure performed on tissues, organs, portion of an organ or cells that have been removed from a living organism, but are studied or manipulated outside the organism in a controlled environment.

The term "decellularized extracellular matrix", in the context of the present invention, refers to the extracellular matrix (ECM) of a tissue, organ or portion of an organ that has been processed to remove all its cellular components through the decellularization process, leaving behind only the non-cellular structural framework. In this invention, the decellularized extracellular matrix is used as a scaffold to be repopulated with new cells.

The term of "regenerative cells", in the context of the present invention refers to cells with the capability of regeneration of new tissue, preferably endothelial and/or stromal cells. This is done through angiogenesis for endothelial cells and differentiation into other tissue types for stromal cells. Furthermore, the type of endothelial cells will preferably comprise umbilical vein endothelial cells (UVECs) and outgrowth endothelial cells (OECs), and stromal cells selected from the consisting of mesenchymal stem cells (MSCs) and smooth muscle cells (SMCs).

The term of "perfusion", in the context of the present invention refers to the process of delivering a controlled flow of fluids (such as culture media, oxygenated solutions, and growth factors) through or around the cells at specific pressures to encourage them to adhere, integrate, and survive within the target organ or portion of an organ.

The term of "media", in the context of the present invention refers to the nutrient-rich solutions or substances used to support the growth, survival, and development of cells, tissues, or in vitro. In the present invention, the term "proliferation media" is used, to refer to the media to promote the rapid growth and division of cells. Furthermore, in the present invention, the term "differentiation media" is used, to refer to media used to induce cells, particularly stem or progenitor cells, to differentiate into specific cell types. It is also referred to with the term "maturation media". Lastly, in the present invention, the term "adequate media" is used, to refer to the cell culture medium that is specifically formulated to provide the necessary nutrients, growth factors, and environmental conditions required for the optimal growth, survival, proliferation, and function of a particular cell type that is being referred to.

The term of "re-cellularize", in the context of the present invention refers to the process of seeding new cells onto or into a decellularized extracellular matrix (such as an organ or portion of an organ).

The term of "cannulate", in the context of the present invention refers to the process of inserting a cannula, which is a thin, flexible tube, into a body cavity, duct, vessel, or other anatomical structure. In the present invention, the term "cannulated portion" is used, to refer to the organ or portion of an organ that was cannulated at one or more cavities, vessels and or ducts.

The term of "preconditioning", in the context of the present invention refers to the process of preparing or conditioning the organ or portion of an organ in advance to optimize its performance or functionality for the in-vivo transplant.

The term of "in-vivo transplant", in the context of the present invention refers to the procedure where the re-cellularized extracellular organ or of a portion of an organ are transplanted directly into a living organism.

The term of "injection routes", in the context of the present invention refers to the various pathways or methods used to introduce cells, fluids, or substances into a scaffold during the re-cellularization process of the de-cellularized matrix of an organ or portion of an organ.

The term of "hepatic parenchyma", in the context of the present invention refers to the functional tissue of the liver, primarily made up of hepatocytes, which are the liver cells responsible for performing the essential functions of the liver.

The term of "physiological pressures", in the context of the present invention refers to the replication of the natural pressure conditions (such as blood pressure or fluid flow pressure showed in vivo) within bioreactors and/or during recellularization of the scaffold. These pressures vary depending on the organ.

The term of "regenerative pressures", in the context of the present invention refers to the mechanical forces or pressure conditions applied to cells to promote growth, differentiation, and tissue regeneration. These pressures are designed to mimic the natural mechanical environment that cells would experience in the body and to stimulate biological responses that aid in tissue formation. These pressures vary depending on the organ.

The term of "bioreactor", in the context of the present invention refers to a specialized device or system that provides a controlled environment for the cells to proliferate and differentiate, under specific conditions.

### Description

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

Tissue generation is a rapidly advancing field within biomedical engineering, aiming to create living tissues to repair, replace, or regenerate damaged structures in the human body. It involves the combination of cells, biomaterials (such as decellularized or synthetic scaffolds), and biological factors to promote the formation of functional tissues. Significant progress has been made in areas such as skin and cartilage regeneration, and research is ongoing to address more complex organs like the heart, liver, and kidneys.

Despite these advances, one of the main technical challenges in tissue generation is the difficulty of creating fully functional tissue. This problem includes the inability to precisely replicate the complex architecture, vascularization (blood vessel systems), and cellular interactions required for the tissue to not only survive but also function properly within the body. For instance, in thicker or more complex tissues, revascularization is often insufficient to provide adequate oxygen and nutrient supply, limiting long-term tissue viability.

In order to solve this problem, the authors of the present invention tested the use of two or more cells, preferably regenerative cells, in-vitro, within a proliferation media with the use of different seeding and preconditioning pressures. Furthermore, they explored the possibility of cannulating the organ or portion of an organ for the cells to be properly perfused. The results proved that the change between high and low pressures, together with the use of different types of regenerative cells that are perfused, made it possible to cells to proliferate within the de-cellularized organ and continue functioning in-vivo after transplantation. Moreover, this use of alternating pressures allowed the inventors to specifically target different vascular structures with different diameters (higher perfusion pressures targeted smaller diameter vessels, while low pressures target larger vessels).

In view of the above results, the present invention provides a functional vascular tree within an organ or portion of an organ, being suitable for its use in transplant for a subject in need thereof.

In view of the above, in a **first aspect,** the present invention relates to an *ex vivo* method of re-cellularizing a perfusion decellularized extracellular matrix of an organ or of a portion of an organ. Said method, comprises providing said decellularized extracellular matrix of an organ or of a portion of an organ, and contacting said decellularized extracellular matrix of an organ or of a portion of an organ with a population of cells, preferably regenerative cells, under conditions in which said population of cells, preferably regenerative cells, engraft, multiply and/or differentiate within and on said decellularized extracellular matrix portion, wherein said conditions comprises:
a) injecting or perfusing the cells into the decellularized extracellular matrix at a pressure of between 1 and 200 mmHg so as to engraft the cells in the said decellularized extracellular matrix;
b) expand the cells by using a proliferation media; and
c) differentiate the cells by using a maturation media.

Each of the steps are explained in detail below:
Step a) comprises or consists of injecting or perfusing the cells into the decellularized extracellular matrix at a pressure of between 1 and 200 mmHg so as to engraft the cells in the said decellularized extracellular matrix.

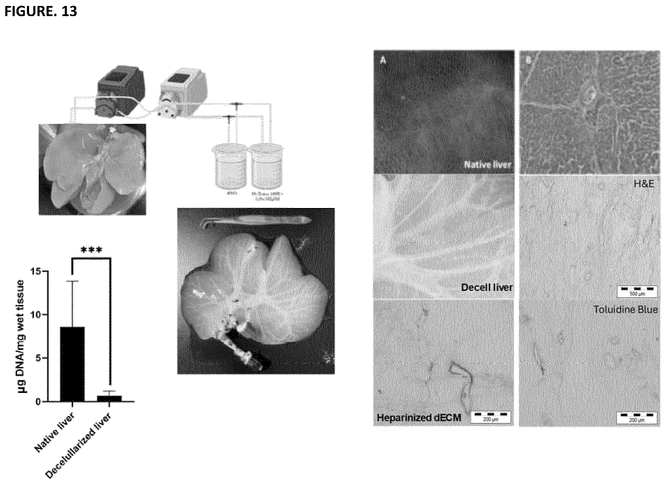

Said step comprises injecting or perfusing cells into the decellularized extracellular matrix so they are able to engraft. Said decellularized extracellular matrix has been previously decellularized and heparinized, in order to avoid blood clots and increase biocompatibility. The decellularization process and further heparinization is done as explained in the examples (See Figure 13).

In an embodiment, the decellularized extracellular matrix of an organ or of a portion of an organ is the liver, preferably the liver of a pig, bovine, sheep, canine or human being.

In another embodiment of the first aspect of the invention, said decellularized extracellular matrix of an organ or of a portion of an organ is re-cellularized with at least two different types of cells, preferably regenerative cells. In a preferred embodiment, the cells are endothelial cells selected from the list consisting of umbilical vein endothelial cells (UVECs) and outgrowth endothelial cells (OECs), and stromal cells selected from the consisting of mesenchymal stem cells (MSCs) and smooth muscle cells (SMCs). These types of cells were chosen since, in order to reach complete repopulation of the scaffold, angiogenic cell types with high proliferative capacity should be used. It is important to note that in this invention when we are referring to regenerative cells, we refer to only MSCs and OECs regenerative capacities, having MSCs broader differentiation potential.

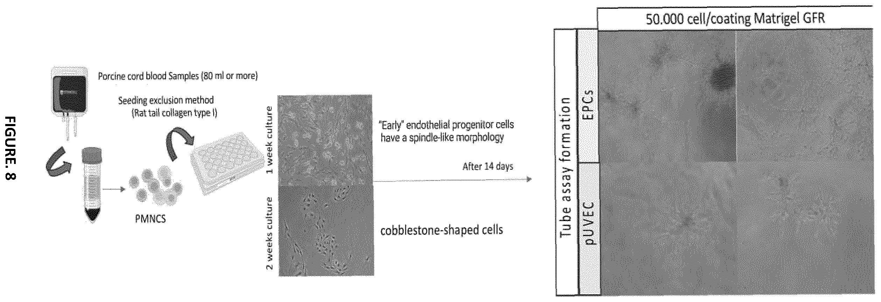

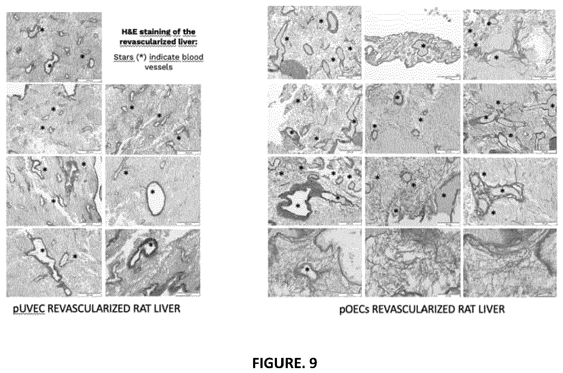

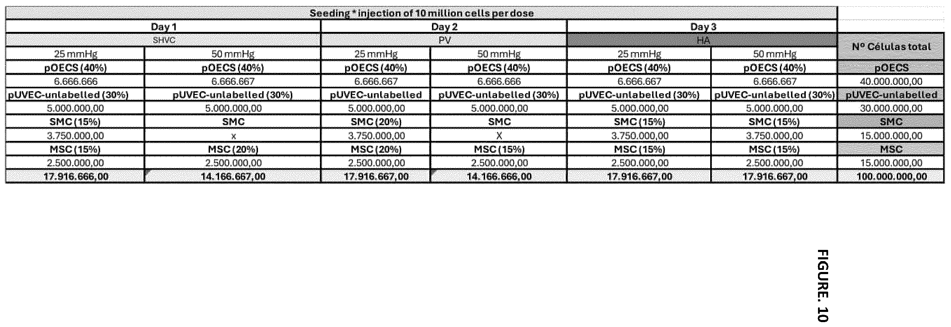

Talking about angiogenic properties of the previously mentioned cells, as an expert in the topic would know, UVECs and OECs have directly angiogenic properties, since endothelial cells are directly involved in angiogenesis. MSCs cells are indirectly angiogenic, they support angiogenesis through the secretion of pro-angiogenic factors. Lastly, SMCs play a supportive role in angiogenesis, contributing to the stabilization and functional maturation of vessels by forming smooth muscle layers that confer contractile properties. Up-scaling from rat to porcine liver required further exploring the potential of porcine OECs (pOECs) in revascularization of the decellularized matrix. As explained in the examples and in Figures 8 and 9, the proliferative capacities of pOECS in vitro models were superior to those from porcine UVECs (pUVECs). The introduction of pOECS allowed for a reduction in the total number of cells seeded, due to greater angiogenic potential. Adjusted cellular percentages appear in Figure 10.

In an embodiment, step a) of the method comprises cannulating said organ at one or more cavities, vessels, and/or ducts, thereby producing a cannulated portion; and perfusing said cannulated portion with a seeding medium comprising the cells, preferably the regenerative cells so as to provide a re-cellularized organ or of a portion of an organ. The cannulation allows direct access to the vascular network of the organ, enabling the engineered cells to be distributed through existing blood vessels (devoid of cells) or other channels, mimicking the way blood naturally flows through the tissue. In a preferred embodiment, the organ is cannulated at the suprahepatic vena cava (SHVC), the hepatic artery (HA) and portal vein (PV) of the decellularized liver. In some occasions, it could also be cannulated at the infrahepatic vena cava instead of at the suprahepatic vena cava.

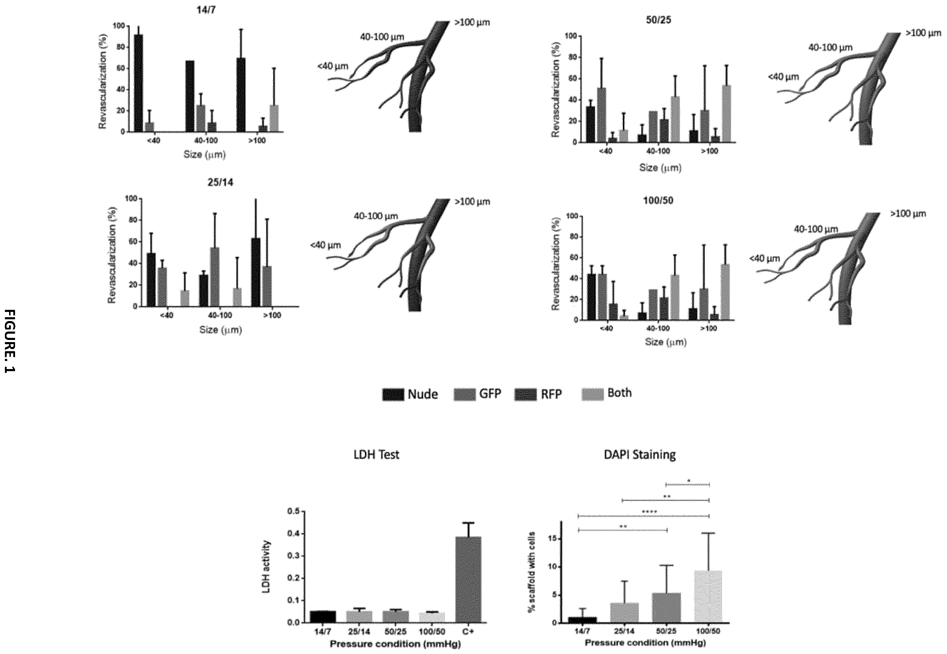

In an embodiment of the first aspect, the cells are injected or perfused at a pressure of between 1 and 200 mmHg, preferably between 5 and 150 mmHg, more preferably between 10 and 100 mmHg, even more preferably between 25 and 50 mmHg. This pressure is used to engraft the cells in the decellularized extracellular matrix. The optimal seeding pressure for rat liver scaffolds were tested, using two different methods: the continuous method and the multi-step method. In the continuous method cells are injected directly into the media and there is a pressure change after 4 hours, while in the multi-step method the cells are injected in the scaffold, with low and high pressure separated by 15 minutes between steps. Data collected from DAPI staining, as explained in the examples and in Figure 1, revealed that the higher-pressure combination produced the highest cell engraftment within the scaffold. Therefore, the final pressures used for seeding are preferably 50-25 mmHg in a multi-step seeding condition.

The purpose of applying a pressure of between 1 and 200 mmHg is to induce specific cell penetration (higher perfusion pressures targeted smaller diameter vessels, while low pressures target larger vessels), so that the cells can easily engraft throughout the whole vascular tree. These pressures are applied in the same bioreactor, to fully revascularize a decellularized liver scaffold.

In an embodiment, the cells are preferably endothelial cells selected from the list consisting of umbilical vein endothelial cells (UVECs) and outgrowth endothelial cells (OECs), and stromal cells selected from the consisting of mesenchymal stem cells (MSCs) and smooth muscle cells (SMCs), and in step a) each cell type is divided for each of the following injection routes PV, SHVC and HA and injected or perfused in each of those injection routes at a concentration of between 1x10⁶ to 1x10⁸ during a time interval of 1 to 3 days. In a preferred embodiment, wherein said administration is carried out by injecting per injection route, in one or more injections per day, the total or a portion of the total amount of cells during a time interval of 1 to 3 days, at decreasing pressures within the range of between 150 and 10 mmHg. In a preferred embodiment, wherein in day 1 about 33% of the total amount of UVEC cells, 33% of the total amount of OECs cells, 50% of the total amount of MSC cells and 30% of the total amount of SMC cells are administered at a pressure of about 100 mmHg, wherein in day 2 about 33% of the total amount of UVEC cells, 33% of the total amount of OECs cells, 30% of the total amount of MSC cells and 40% of the total amount of SMC cells are administered at a pressure of about 50 mmHg, and wherein in day 3 the rest of the cells of the total amount of cells are administered at a pressure of about 25 mmHg.

In an embodiment of the first aspect, the cells are preferably endothelial cells selected from the list consisting of umbilical vein endothelial cells (UVECs) and outgrowth endothelial cells (OECs), and stromal cells selected from the consisting of mesenchymal stem cells (MSCs) and smooth muscle cells (SMCs), and each day the amount of cells are administered in at least 2-3 injections, and wherein each day one or more extra recirculation injections at the seeding pressure with basal medium are carried out to ensure proper cell penetration. In another preferred embodiment, wherein the total amount administered per each cell type is divided for each of the following injection routes PV, SHVC and HA in the following proportions, about 30% of the total amount of UVEC and OECS are administered via PV, about 50% of the total amount of endothelial cells are administered via SHVC, about 20% of the total amount of endothelial cells are administered via HA, about 40% of the total amount of MSC are administered via PV, about 40% of the total amount of stromal cells are administered via SHVC, and about 20% of the total amount of stromal cells are administered via HA. Also, about 30% of the total amount of SMC are administered via PV, about 30% of the total amount of stromal cells is administered via SHVC, and about 40% of the total amount of stromal cells are administered via HA.

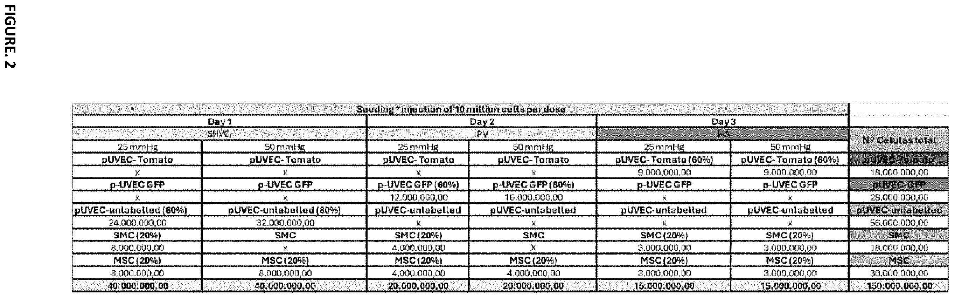

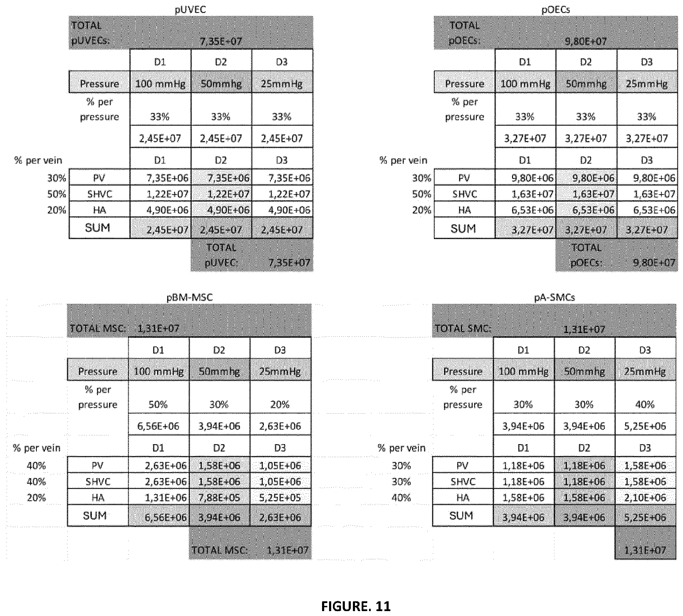

Figure 2 shows the seeding cells schedule for a rat liver, the percentage of cells that are injected, the pressure and the total cell number and Figure 11 show the schedule, percentage, pressures applied and total cell seeding number of a porcine liver.

Step b) comprises or consists of expanding the cells by using a proliferation media.

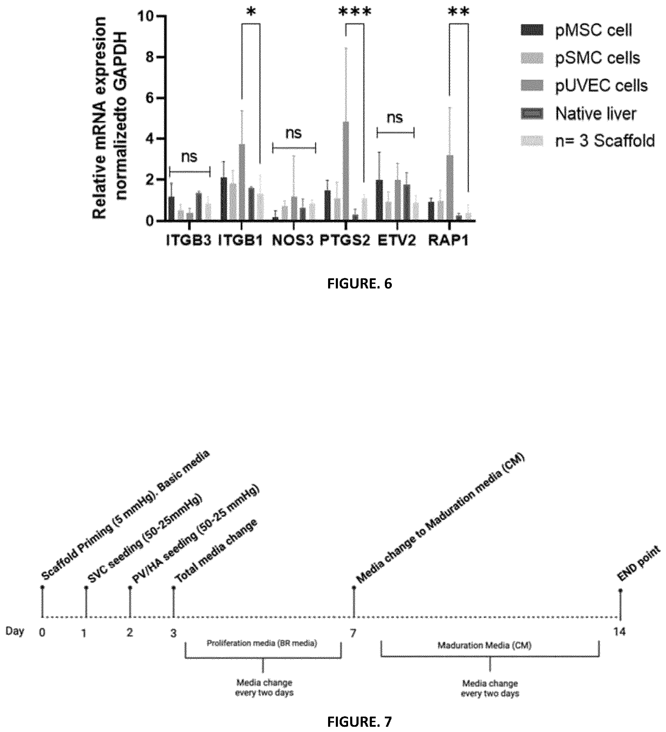

Said step comprises expanding the cells by using a proliferation media. Said proliferation media, as explained in the examples, was used first during cell seeding and then during the first days of bioreactor cell culture. The exact composition of this medium was: DMEM/F12 (Sigma D8062) and MCBD131 (Gibco 10372019), supplemented with penicillin/streptomycin/amphotericin-b (Sigma A5955, 1%), L-glutamine (Sigma G7513, 2 mM), fetal bovine serum (Gibco 10270106, 5%), insulin (Sigma I9278, 5 µg/ml), transferrin (Sigma T0665, 10 µg/ml), VEGF-A (Prepotech 100-20, 50 ng/ml), EGF (Peprotech AF-100-15, 40 ng/ml), FGF2 (Peprotech 100-18B, 40ng/ml) and IGF1 (Peprotech 100-11R3, 40 ng/ml). This can be seen in Figure 7.

Step c) comprises or consists of differentiating the cells by using a differentiation media.

Said step comprises differentiating the cells by using a maturation media. It is important to note that in this invention, the terms differentiation and maturation media are the same. Said differentiation media, as explained in the examples, is used after the 4-7^{th} day of bioreactor culture. It is a mixture of vascular induction medium (richer in pro-angiogenic factors such as VEGF, FGF-2, IGF-1, and Ang-2) and blood vessel maturation medium (richer in pro-maturation factors such as Ang-1, TFG-β1, and PDGF-BB). The exact composition of this medium was: DMEM/F12 (Sigma D8062) and MCBD131 (Gibco 10372019), supplemented with penicillin/streptomycin/amphotericin-b (Sigma A5955, 1%), L-glutamine (Sigma G7513, 1%), fetal bovine serum (Gibco 10270106, 1%), insulin (Sigma I9278, 5 µg/ml), transferrin

(Sigma T0665, 10 µg/ml), VEGFA (Peprotech 100-20, 25 ng/ml), FGF2 (Peprotech 100-18B, 20ng/ml), IGF1 (Peprotech 100-11R3, 20 ng/ml), Ang-2 (Peprotech 130-07, 50 ng/ml), Ang-1 (Peprotech 130-06 25 ng/ml), TGF-β1 (Peprotech 100-21 0,05 ng/ml) and PDGF-BB (Peprotech 100-14B 5ng/ml).

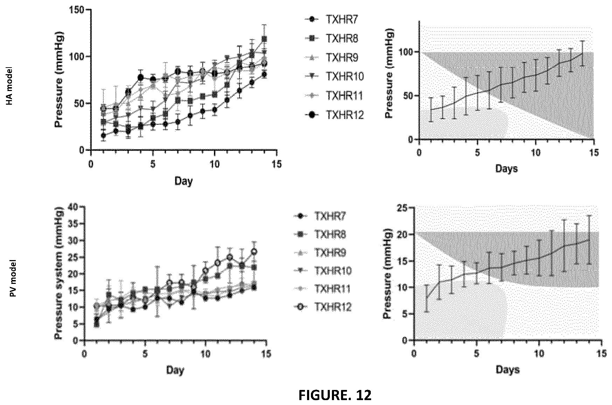

In an embodiment of the first aspect, the re-cellularized extracellular organ or of a portion of an organ derived from step c) is preconditioned, at physiological pressures, preferably for a duration of at least 1 to 14 days, prior to in-vivo transplant by using an adequate media. This mechanical adaptation (See Figure 12) uses the physiological pressures of the target organ being transplanted for both vein and artery.

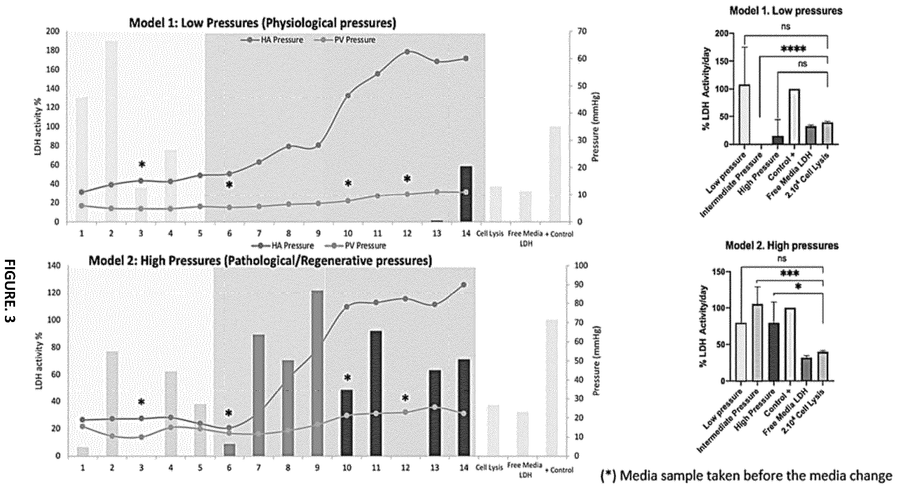

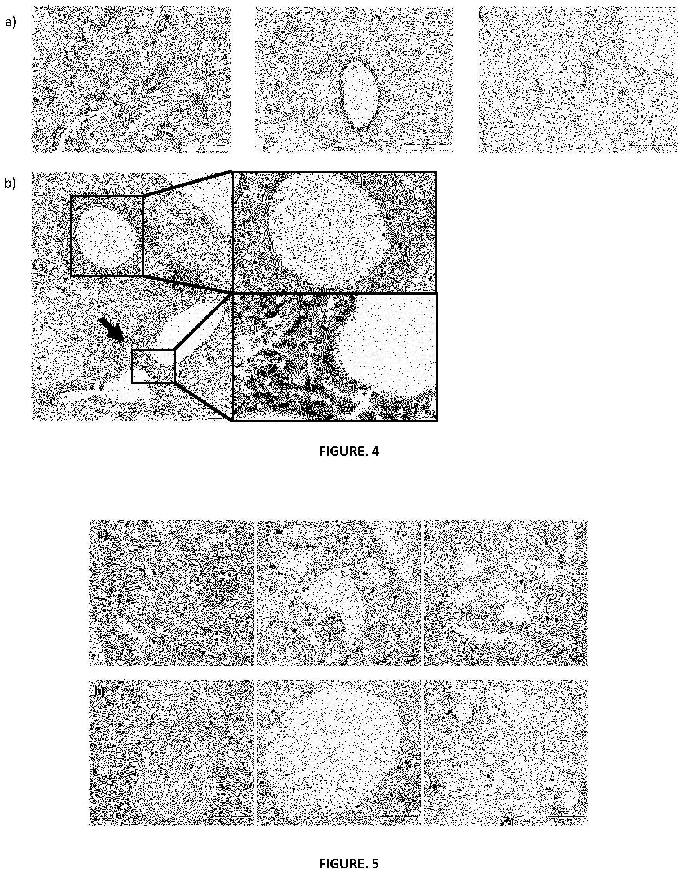

For the proliferation and maturation in steps b) and c), the pressures of the dynamic system during the 14-days vasculogenesis process were studied. As explained in the examples, this monitoring is essential so that the cells do not suffer mechanical damage while adapting to the shear stress that they will experience in vivo due to blood circulation. The results obtained after quantifying the LDH concentration in the medium (See Figure 3) on different days at both high and low pressures, along with the histological results (See Figures 4 and 5), led to the establishment of the low-pressure maintenance model. In the high-pressure model there was a high cell-death (See Figure 3).

In a **second aspect,** the present invention provides a functional vascular tree within a re-cellularized extracellular matrix of an organ or of a portion of an organ obtained or obtainable from the method defined in the first aspect or any of its embodiments.

Preferably, the re-cellularized extracellular matrix of an organ or of a portion of an organ obtained or obtainable from the method defined in the first aspect or any of its embodiments is from a liver organ.

Preferably, the functional vascular tree within a re-cellularized extracellular matrix of an organ or of a portion of an organ obtained or obtainable from the method defined in the first aspect or any of its embodiments can be perfused.

Preferably, the functional vascular tree of the second aspect is obtained or obtainable from a method comprising the steps of:
a) injecting or perfusing the cells into the decellularized extracellular matrix at a pressure of between 1 and 200 mmHg so as to engraft the cells in the said decellularized extracellular matrix;
b) expand the cells by using a proliferation media; and
c) differentiate the cells by using a differentiation media.

Preferably said decellularized extracellular matrix of an organ or of a portion of an organ is re-cellularized with at least two different types of regenerative cells.

Preferably, step a) of the method comprises cannulating said organ at one or more cavities, vessels, and/or ducts, thereby producing a cannulated portion; and perfusing said cannulated portion with a seeding medium comprising the regenerative cells so as to provide a re-cellularized organ or of a portion of an organ.

Preferably, the re-cellularized extracellular organ or of a portion of an organ derived from step c is preconditioned, at physiological pressures, preferably for a duration of at least 1 to 14 days, prior to in-vivo transplant by using an adequate media.

Preferably, said decellularized extracellular matrix of an organ or of a portion of an organ is the liver, preferably the liver of a pig, bovine, sheep, canine or human being. Preferably, the method comprises cannulating said organ at the suprahepatic vena cava(SHVC), the hepatic artery (HA) and portal vein (PV).

Preferably, the cells are endothelial cells selected from the list consisting of Umbilical Vein Endothelial Cells (UVECs) and Outgrowth Endothelial Cells (OECs) and stromal cells selected from the consisting of Mesenchymal Stem Cells (MSCs) and Smooth Muscle Cells (SMCs).

Preferably, the cells are preferably endothelial cells selected from the list consisting of umbilical vein endothelial cells (UVECs) and outgrowth endothelial cells (OECs), and stromal cells selected from the consisting of mesenchymal stem cells (MSCs) and smooth muscle cells (SMCs), and in step a) each cell type is divided for each of the following injection routes PV, SHVC and HA and injected or perfused in each of those injection routes at a concentration of between 1x10⁶ to 1x10⁸ during a time interval of 1 to 3 days. Preferably, said administration is carried out by injecting per injection route, in one or more injections per day, the total or a portion of the total amount of cells during a time interval of 1 to 3 days, at decreasing pressures within the range of between 150 and 10 mmHg. Preferably, the cells are administered during a time interval of three days, wherein in day on day 1 about 33% of the total amount of UVEC cells, 33% of the total amount of OECs cells, 50% of the total amount of MSC cells and 30% of the total amount of SMC cells are administered at a pressure of about 100 mmHg, wherein in day 2 about 33% of the total amount of UVEC cells, 33% of the total amount of OECs cells, 30% of the total amount of MSC cells and 40% of the total amount of SMC cells are administered at a pressure of about 50 mmHg, and wherein in day 3 the rest of the cells of the total amount of cells are administered at a pressure of about 25 mmHg.

Preferably, each day the amount of cells are administered in at least 2-3 injections, and wherein each day one or more extra recirculation injections at the seeding pressure with basal medium are carried out to ensure proper cell penetration.

Preferably, the total amount administered per each cell type is divided for each of the following injection routes PV, SHVC and HA in the following proportions, about 30% of the total amount of UVEC and OECS are administered via PV, about 50% of the total amount of endothelial cells are administered via SHVC, about 20% of the total amount of endothelial cells are administered via HA, about 40% of the total amount of MSC are administered via PV, about 40% of the total amount of stromal cells are administered via SHVC, and about 20% of the total amount of stromal cells are administered via HA. Also, about 30% of the total amount of SMC are administered via PV, about 30% of the total amount of stromal cells is administered via SHVC, and about 40% of the total amount of stromal cells are administered via HA.

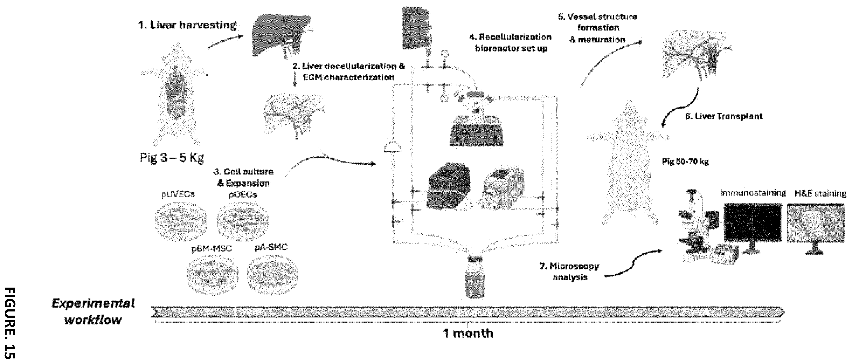

In a **third aspect,** the present invention provides the use in organ transplantation for a subject in need thereof as defined in the first or second aspects. In an embodiment, the transplant is performed with a revascularized decellularized liver, in which the liver scaffold was procured from a healthy pig to another healthy pig that will be immunosuppressed, as can be visualized in Figure 15.

The following clauses are also encompassed by the present invention:
1. An *ex vivo* method of re-cellularizing a perfusion decellularized extracellular matrix of an organ or of a portion of an organ, comprising providing said decellularized extracellular matrix of an organ or of a portion of an organ, and contacting said decellularized extracellular matrix of an organ or of a portion of an organ with a population cells, preferably regenerative cells, under conditions in which said population of cells, preferably regenerative cells, engraft, multiply and/or differentiate within and on said decellularized extracellular matrix portion, wherein said conditions comprises:
   a. injecting or perfusing the cells into the decellularized extracellular matrix at a pressure of between 1 and 200 mmHg so as to engraft the cells in the said decellularized extracellular matrix;
   b. expand the cells by using a proliferation media; and
   c. differentiate the cells by using a differentiation media.
2. The method according to clause 1, wherein said decellularized extracellular matrix of an organ or of a portion of an organ is re-cellularized with at least two different types of regenerative cells.
3. The method according to any one of the previous clauses, wherein step a) of the method comprises cannulating said organ at one or more cavities, vessels, and/or ducts, thereby producing a cannulated portion; and perfusing said cannulated portion with a seeding medium comprising the regenerative cells so as to provide a re-cellularized organ or of a portion of an organ.
4. The method according to clauses 1 to 3, wherein the re-cellularized extracellular organ or of a portion of an organ derived from step c is preconditioned, at physiological pressures, preferably for a duration of at least 1 to 14 days, prior to in-vivo transplant by using an adequate media.
5. The method according to clauses 1 to 4, wherein said decellularized extracellular matrix of an organ or of a portion of an organ is the liver, preferably the liver of a pig, bovine, sheep, canine or human being.
6. The method according to clause 5 wherein the method comprises cannulating said organ at the Suprahepatic Vena Cava (SHVC), the Hepatic Artery (HA), and the Portal Vein (PV).
7. The method according to any one of clauses 1 to 6, wherein the cells are endothelial cells selected from the list consisting of Umbilical Vein Endothelial Cells (UVECs) and Outgrowth Endothelial Cells (OECs) and stromal cells selected from the consisting of Mesenchymal Stem Cells (MSCs) and Smooth Muscle Cells (SMCs).
8. The method according to clause 7, wherein in step a) each cell type is divided for each of the following injection routes PV, SHVC and HA and injected or perfused in each of those injection routes at a concentration of between 1x10⁶ to 1x10⁸ during a time interval of 1 to 3 days.
9. The method according to clause 8, wherein said administration is carried out by injecting per injection route, in one or more injections per day, the total or a portion of the total amount of cells during a time interval of 1 to 3 days, at decreasing pressures within the range of between 150 and 10 mmHg.
10. The method according to clause 9, wherein the cells are administered during a time interval of three days, wherein on day 1 about 33% of the total amount of UVEC cells, 33% of the total amount of OECs cells, 50% of the total amount of MSC cells and 30% of the total amount of SMC cells are administered at a pressure of about 100 mmHg, wherein in day 2 about 33% of the total amount of UVEC cells, 33% of the total amount of OECs cells, 30% of the total amount of MSC cells and 40% of the total amount of SMC cells are administered at a pressure of about 50 mmHg, and wherein in day 3 the rest of the cells of the total amount of cells are administered at a pressure of about 25 mmHg.
11. The method, according to clause 10, wherein each day the amount of cells is administered in at least 2-3 injections, and wherein each day one or more extra recirculation injections at the seeding pressure with basal medium are carried out to ensure proper cell penetration.
12. The method according to any one of clauses 8 to 11, wherein the total amount administered per each cell type is divided for each of the following injection routes PV, SHVC and HA in the following proportions, about 30% of the total amount of UVEC AND OECS are administered via PV, about 50% of the total amount of endothelial cells are administered via SHVC, about 20% of the total amount of endothelial cells are administered via HA, about 40% of the total amount of MSC are administered via PV, about 40% of the total amount of stromal cells are administered via SHVC, and about 20% of the total amount of stromal cells are administered via HA. Also, about 30% of the total amount of SMC are administered via PV, about 30% of the total amount of stromal cells is administered via SHVC, and about 40% of the total amount of stromal cells are administered via HA.

The invention is described below by the following examples, which must be considered as merely illustrative and in no case limiting of the scope of the present invention.

### Examples

### Materials and methods

### Cell culture media

The culture media used at each stage of the bioreactor were as follows (See Figure 7):
- **Basic Medium** (priming): DMEM/F12 (Sigma D8062) supplemented with penicillin/streptomycin/amphotericin-b (Sigma A5955, 1%), L-glutamine (Sigma G7513, 2 mM), fetal bovine serum (Gibco 10270106, 10%).
- **Proliferation Media** (BM): was used first during cell seeding and then during the first days of bioreactor cell culture. The exact composition of this medium was: DMEM/F12 (Sigma D8062) and MCBD131 (Gibco 10372019), supplemented with penicillin/streptomycin/amphotericin-b (Sigma A5955, 1%), L-glutamine (Sigma G7513, 2 mM), fetal bovine serum (Gibco 10270106, 5%), insulin (Sigma I9278, 5 µg/ml), transferrin (Sigma T0665, 10 µg/ml), VEGF-A (Prepotech 100-20, 50 ng/ml), EGF (Peprotech AF-100-15, 40 ng/ml), FGF2 (Peprotech 100-18B, 40ng/ml) and IGF1 (Peprotech 100-11 R3, 40 ng/ml).
- **Maturation Media** (CM): used until the end of the experiment. It is a mixture of vascular induction medium (richer in pro-angiogenic factors such as VEGF, FGF-2, IGF-1, and Ang-2) and blood vessel maturation medium (richer in pro-maturation factors such as Ang-1, TFG-β1, and PDGF-BB). The exact composition of this medium was: DMEM/F12 (Sigma D8062) and MCBD131 (Gibco 10372019), supplemented with penicillin/streptomycin/amphotericin-b (Sigma A5955, 1%), L-glutamine (Sigma G7513, 1%), fetal bovine serum (Gibco 10270106, 1%), insulin (Sigma I9278, 5 µg/ml), transferrin (Sigma T0665, 10 µg/ml), VEGFA (Peprotech 100-20, 25 ng/ml), FGF2 (Peprotech 100-18B, 20ng/ml), IGF1 (Peprotech 100-11R3, 20 ng/ml), Ang-2 (Peprotech 130-07, 50 ng/ml), Ang-1 (Peprotech 130-06 25 ng/ml), TGF-β1 (Peprotech 100-21 0,05 ng/ml) and PDGF-BB (Peprotech 100-14B 5ng/ml).

### Carbachol administration protocol

1. 0.1 mM Carbachol.
   a. Administered for 3 hours with data collection during this period.
   b. First Medium Change: After 3 hours, the medium was changed, followed by 1 hour of recirculation with the new medium.
   c. Second Medium Change: The medium was changed again, followed by 30 minutes of recirculation.
2. 1 mM Carbachol.
   a. Administered after the second medium change, with 3 hours of data collection.
3. Fixation: After the data collection for the 1 mM concentration, the scaffold was fixed in 4% paraformaldehyde (PFA) overnight and then paraffin-embedded for histological analysis.

### Decellularization Pressures scale-up and scaffold heparinization.

Both rat and pig scaffolds were decellularized using (1% Triton X-100 or 1% SDS respectively) + 0.1% NH₄OH and washed with deionized water. Afterward, they were stored in deionized water and sent for irradiation at 10,000 Gy for sterilization.

The decellularization protocol followed in rats was scaled up for the decellularization of porcine livers weighing 112 g ± SD 17.69. Since the internal pressure that the scaffold can withstand (≤ 100 mmHg) decreases as the cellular content is removed, the decellularization flow applied to the scaffold increases from stage 1 to stage 5.

Rat liver decellularization:
- PV:
   ∘ Stage 1-2: 2ml/min
   ∘ Stage 3-4: 2,5 ml/min
   ∘ Stage 5: 2,5-3 ml/min
- HA:
   ∘ Stage 1-2: 1 ml/min
   ∘ Stage 3-4: 1,5 ml/min
   ∘ Stage 4-5: 1,5-2 ml/min

### EPA technique.

Heparin was immobilized on the DLS via EPA according to the method modified by Larm et al. [1]. Firstly, sodium heparin was dissolved in distilled water (1g/300mL) at approximately 0°C. The dissolved heparin was partially depolymerized by adding sodium nitrite (10mg) to the solution (pH 2.7, adjusted with 1N HCl) and then stirred at 0°C for 2h. Subsequently, the pH of the solution was adjusted to 7.0 with 1N NaOH. The solution containing partially depolymerized heparin (Diluted to the concentration of heparin 3.3 g/L) with NaBH₃CN (0.01 mg/mL) and NaCl (0.15M) at pH3.5 were perfused into DLSs through PV at 37°C, and then recycled at 100mL/min for 4h. Nitrite-depolymerized heparin can be immobilized via covalent end-point attachment to the DLS. (See Figure 13)

Each heparin-immobilized DLS was sampled from 12 sites and cut into 300-µm thick sections with a diameter of 1cm and used in the quantification of heparin immobilized on the DLS as determined by Smith et al. [2]

The non-heparinized and heparinized DLS were stained with 1% (w/v) aqueous Toluidine Blue (TB).

### Results and discussion

### Example 1. Determination of optimal seeding pressure for rat liver scaffolds.

To evaluate the optimal seeding pressure for rat liver scaffolds, two cell seeding methods (150 x10⁶ cell/condition) were tested on decellularized rat scaffolds (continuous method and multi-step method) using transfected GFP-endothelial cells (GFP-UVECs) and Td-Tomato mesenchymal cells transfected (Td-Tomato-BM-MSC) in 3-day recellularization bioreactors.

These results revealed that the continuous method (cells injected directly in the media and pressure change after 4h) resulted in lower cell engraftment than the multi-step method (cells injected in the scaffold, low and high pressure separated by 15 minutes between steps). Therefore, the multi-step method was used in the remaining experiments. Within this last-mentioned method, different seeding pressures were evaluated (14/7, 25/14, 50/25, 100/50 mmHg). Statistical analyses determined that there were no significant differences between the four groups of pressure (P value > 0,05). This ensured that even the highest pressures used did not affect cell viability in the bioreactor system.

However, data collected from DAPI staining of the different scaffolds seeded at different pressure combinations revealed that the higher-pressure combination condition used for cell delivery produced the highest cell engraftment within the scaffold. This was statistically significant when compared with the other conditions (p<0,05). Additionally, seeding conditions at 50/25 and 100/50 mmHg showed very similar patterns of cell distribution, where large and intermediate vessels were surrounded by both RFP- and GFP-labeled cells, while smaller vessels were predominantly lined with GFP-labeled cells. This showed that to induce specific cell penetration (higher perfusion pressures targeted smaller diameter vessels, while low pressures targeted larger vessels), so that the cells can easily engraft throughout the whole vascular tree. These pressures are applied in the same bioreactor to fully revascularize a decellularized liver scaffold. (See Figure 1)

Therefore, the established optimal conditions for high and low-pressure cell seeding in decellularized rat scaffolds were 50-25 mmHg in a multi-step seeding condition for further experiments.

### Example 2. Angiogenic cell type for re-cellularization of rat decellularized scaffolds.

In this experiment, the different parts of the hepatic vascular tree are reconstructed: The arterial and venous systems (whose main components are portal triads (HA and PV), central veins (CV), and parenchymal sinusoids). For this purpose, a new 3 days-seeding regimen was established, using a total of 150x10⁶ cells/bioreactor (main cell types that comprise the native physiology of the vascular tree: endothelial cells (GFP (only by PV liver vessel), Td-Tomato (only by HA liver vessel), and unlabeled cells (only by SHVC liver vessel)), aortic muscle cells (A-SMC), and bone marrow mesenchymal stem cells (BM-MSC)). The seeding percentages of each cell type varied depending on the target vascular tree (HA, PV, or CV) (See Figure 2) as well as the targeted vascular diameter by the injection pressure: high seeding pressure (50 mmHg) for small and medium, or low seeding pressure (25 mmHg) for large diameter vessels.

No more than 10 million cells should be resuspended in 8-10 ml injection. Since the cell numbers were higher in all conditions, 30 minutes were allowed between injections at the same pressure (e.g., SHVC 50 mmHg Injection 1 - 30 min - Injection 2 - 30 min - Injection 3 - 30 min - Injection 4), and 30min start/stop recirculation were allowed between different pressures (e.g., SHVC 50 mmHg Injection 4 - 30 min media recirculation at 50 mmHg - 30 min static media- SHVC 25 mmHg Injection 1). Another 30-minute start/stop recirculation at 25 mmHg was allowed before establishing overnight maintenance circulation at 2 ml/min for SHVC, 1 ml/min for PV, and 0,1 ml/min for HA.

The solution lies in finding an angiogenic cell type with high proliferative capacity that can stimulate the formation of tip cells and propagate throughout the hepatic parenchyma.

### Example 3. Testing of high and low pressure maintenance regimens.

To determine the role of sustained and ramping biomechanical stimulation in vascular maturation and function, two different bioreactor maintenance regimens were tested after cell seeding injection (25-50 mmHg).

### High-pressure regimen (n=2) →Pathological/regenerative pressures:

- PV: initial pressure of 10mmHg for 7 days, followed by an increase of 3mmHg/day for 5 days until it reaches 20mmHg, pressure which will be maintained until day 14.
- HA: 20mmHg 7 days, followed by an increase in pressure of 20mmHg/day until it reaches approximately 80mmHg pressure which will be maintained until day 14.

### Low-pressure regimen (n=2) →Physiological pressures:

- PV: initial pressure of 5 mmHg for 7 days, followed by increase 1mmHg/day for 7 days until it reaches approximately 10mmHg, pressure which will be maintained until day 14
- HA: 10mmHg 7 days, followed by an increase in pressure of 10mmHg/day until it reaches approximately 60mmHg pressure which will be maintained until day 14.

The results obtained after quantifying the LDH concentration in the medium at different days in both models, along with the histological results (H&E and immunofluorescence staining), allowed us to discard the high-pressure maintenance model due to the high cell death that occurred (See Figure 3, Figure 4 and Figure 5). Consequently, the low-pressure maintenance/ramping regimen model was used in further experiments.

To assess the vascular cells not only repopulate the scaffold appropriately but also the different seeded cell percentages in the decellularized scaffold were able to organize into functional vascular structures and mimic native vascular physiology, n=3 bioreactors were set up under the previous seeding conditions. These bioreactors were maintained under a low-pressure maintenance/ramping regimen for 14 days.

### Example 4. Determination of vascular structures functionality in rat decellularized scaffolds.

After observing that the revascularized rat scaffolds exhibited different diameters and cell compositions of vascular structures, whose spatial organization resembles that observed in vivo, the supernatants from these bioreactors (n=3) were analyzed to determine the presence of pro-angiogenic agents derived from the functionality of the endothelial cells: nitric oxide (NO) (absorbance kit) and prostacyclin secretion (ELISA kit). Additionally, it was observed that the 100 nM bradykinin addition to the culture media on day 14 intensified the production of before mentioned molecules suggesting a potential method for further promoting vascular functionality in tissue-engineered constructs. On the other hand, the contractile capacity of the generated vessels was evaluated by the addition of two different concentrations of carbachol (0.1 mM and 1 mM). The increase in pressure recorded by the TAM-A system (3h) was dose-dependent and suggests that the generated vascular structures can increase their intracellular calcium concentration leading to the activation of calmodulin and subsequent activation of myosin light chain kinase (MLCK). MLCK phosphorylates myosin light chains, enabling interaction with actin filaments and resulting in smooth muscle contraction. Although endothelial cells themselves do not contract in response to carbachol, they can influence vascular tone through the release of signaling molecules. Carbachol can stimulate endothelial cells to produce nitric oxide (NO), a potent vasodilator, through a separate pathway involving the activation of endothelial nitric oxide synthase (eNOS).

The 100nM Bradykinin concentration was calculated considering the total volume of media within the bioreactor. On day 14, 5 mL media was withdrawn and used to resuspend the bradykinin and directly added to the scaffold at a flow rate of 1 mL/min through the PV using a syringe pump system. 2 ml media was recollected before and 45 min/ 24h after the addition to determine their effect in PGI2 and NO secretion.

To avoid interference between experiments, the effects of carbachol were assessed on day 15. Both concentrations of carbachol were calculated based on the total volume of the bioreactor. The administration was performed as follows:
- Initial media extraction: 5 mL of media was extracted from the bioreactor.
- Direct Injection: The carbachol solution was injected directly into the bioreactor through the PV using a peristaltic pump at a flow rate of 1 mL/min.

### Example 5. Determination of endothelial gene expression

The mRNA expression levels of different genes involved in angiogenesis were analyzed by semi-quantitative RT-PCR. The expression level of the target gene was normalized to the expression level of the housekeeping gene (GAPDH). (See Figure 6)

### Two-way ANOVA test

- ITGB3: critical in the regulation of angiogenesis, the process by which new blood vessels form from pre-existing ones. It interacts with various ligands, such as vitronectin and fibrinogen, to facilitate endothelial cell migration, proliferation, and survival during angiogenesis.
- ITGB1: ITGB1 binds to ECM proteins such as fibronectin, collagen, and laminin. This binding is crucial for endothelial cell adhesion to the basement membrane and the ECM, maintaining the structural integrity of blood vessels.
- NOS3/eNOS: NOS3-derived NO is crucial for endothelium-dependent vasodilation. It helps maintain vascular tone and ensures adequate blood supply to tissues.
- PTGS2/COX2: PTGS2-derived prostaglandins stimulate angiogenesis (the formation of new blood vessels) by enhancing the expression of angiogenic factors like VEGF (vascular endothelial growth factor).
- ETV2/ER71: ETV2 is crucial for the early specification of endothelial cells from mesodermal progenitors during embryogenesis. It is one of the earliest markers of endothelial cell fate.
- RAP1: is crucial for the inside-out activation of integrins, which are transmembrane receptors that facilitate cell-ECM (extracellular matrix) adhesion. By activating integrins, RAP1 enhances the adhesive properties of endothelial cells, stabilizing their attachment to the ECM.

### Example 6. Exploring pOECs potential in whole-parenchyma revascularization.

Outgrowth endothelial cells (OECs) were isolated from porcine cord blood - at 82 days gestation. Due to the early gestation time, cord blood has a higher concentration of hematopoietic stem cells (HSCs) and endothelial progenitor cells (OECs/EPCs). Therefore, blood pulls (differentiating between male and female fetal piglets) were collected to obtain at least 50 ml of cord blood. The collected cells were seeded in 48-well plates coated with rat tail collagen using an endothelial cell medium (Pelobiotech). The culture medium was maintained until day 3 and thereafter changed every 2 days until endothelial cell colonies appeared by day 14. The obtained colonies were expanded and characterized by flow cytometry and immunofluorescence. It was confirmed that their doubling time (2D plate) and proliferative capabilities in *in vitro* models (both in Matrigel and dECM) were superior to pUVEC cell cultures.

The introduction of pOECs allows for a reduction in the total number of cells seeded due to their more significant angiogenic and proliferative potential. Cellular percentages were adjusted (see Figure 10).

### Example 7. Bioengineering revascularization scale-up. From rat to pig liver vascular tree regeneration

Since a pig scaffold is 10-11 times heavier (mean total wet liver weight was 112 g), than a rat one (mean total wet liver weight was 9,5 g), the cells used in the previous table were scaled up by a factor of 3.5 (Obtaining a higher cell number requires more advanced equipment).

To prevent potential cell aggregation and clogging in the vessels, unlike in rats, the seedings for each route (PV, SHVC, and HA) were performed on separate days at different pressures, from highest to lowest (D1-100 mmHg, D2-50 mmHg, and D3-25 mmHg). An in vitro test was conducted using injection pressures of 50-25 mmHg, and it was insufficient to deliver the cells to the most distal small vessels of the scaffold.

Each cell type was divided for each injection route (PV/SHVC/HA) and seeding day (100, 50, or 25 mmHg). The percentages associated with each pressure injection and administration route aim to mimic the in vivo cellular content (See Figure 8, Figure 9 and Figure 11).

Each route (PV/HA/SHVC) requires 2-3 injections with the cellular content (10 M cells - 4-8 ml each) per day (100/50/25 mmHg), in addition to an extra recirculation injection at the seeding pressure with basal medium to ensure proper cell penetration.

The pressures recorded by the PV and HA pressure sensors were collected over a 14-day period to establish a new ramp model of maintenance pressures to mimic the new physiology-regenerative pressure model and precondition the scaffold for its use in liver transplantation in pigs. As is well known, the physiological portal pressure is within 4-10 mmHg and has a continuous flow, and the hepatic artery displays pressures around 80 mmHg with pulsatile flow. Portal flow in normal individuals is around 1000-1200 mL/min, arterial flow is 300-400 mL/min, and the ratio of portal to arterial flow is 2.5-3.5. However, circuit hemodynamic changes (portal hypertension) occur when a new liver is transplanted. The current knowledge of small for size or "small for flow" syndrome and liver regeneration has shown that higher portal pressure ranging from 10-20 mmHg after partial hepatectomy is conducive to in vivo angiogenesis and liver regeneration. The scaffold is preconditioned for *in vivo* pressures after transplantation using a Low to High pressure ramping regimen.
- Day 1 to 7: Physiological pressures (positive outcome in rats) allowed cell attachment and proliferation within the decellularized scaffold.
   ∘ PV: initial pressure of 5 mmHg, followed by an increase of 1mmHg/day until it reaches approximately 10mmHg, pressure which will be maintained until day 7.
   ∘ HA: 10 mmHg 7 days, followed by an increase in pressure of 10mmHg/day until it reaches approximately 60 mmHg pressure, which will be maintained until day 7.
- Day 7 to 14: Regenerative pressures enabled the cells to adapt to the shear stresses they must withstand *in vivo.*
   ∘ PV: initial pressure of 10 mmHg followed by an increase of 1 mmHg/day for 5 days until it reaches 20 mmHg, which will be maintained until day 14.
   ∘ HA: initial pressure of 60 mmHg, followed by an increase of 10 mmHg/day until it reaches approximately 80 mmHg, which will be maintained until day 14.

The average pressure recorded for each day corresponds to the average of the initial established pressure, after 10 hours, and after 24 hours (See Figure 12).

### REFERENCES

[1]. Larm, O., Larsson, R. & Olsson, P. A new non-thrombogenic surface prepared by selective covalent binding of heparin via a modified reducing terminal residue. Biomater Med Devices Artif Organs 11, 161-173 (1983).
[2]. Smith, P. K., Mallia, A. K. & Hermanson, G. T. Colorimetric method for the assay of heparin content in immobilized heparin preparations. Anal Biochem 109, 466-473 (1980).

## Claims

1. An *ex vivo* method of re-cellularizing a perfusion decellularized extracellular matrix of an organ or of a portion of an organ, comprising providing said decellularized extracellular matrix of an organ or of a portion of an organ, and contacting said decellularized extracellular matrix of an organ or of a portion of an organ with a population of cells, preferably regenerative cells, under conditions in which said population of cells, preferably regenerative cells, engraft, multiply and/or differentiate within and on said decellularized extracellular matrix portion, wherein said conditions comprises:
a. injecting or perfusing the cells into the decellularized extracellular matrix at a pressure of between 1 and 200 mmHg so as to engraft the cells in the said decellularized extracellular matrix;
b. expand the cells by using a proliferation media; and
c. differentiate the cells by using a differentiation media.

2. The method according to claim 1, wherein said decellularized extracellular matrix of an organ or of a portion of an organ is re-cellularized with at least two different types of regenerative cells.

3. The method according to 1 to 2, wherein step a) of the method comprises cannulating said organ at one or more cavities, vessels, and/or ducts, thereby producing a cannulated portion; and perfusing said cannulated portion with a seeding medium comprising the regenerative cells so as to provide a re-cellularized organ or of a portion of an organ.

4. The method according to claims 1 to 3, wherein the re-cellularized extracellular organ or of a portion of an organ derived from step c is preconditioned at physiological pressures, preferably for a duration of at least 1 to 14 days, prior to in-vivo transplant by using an adequate media.

5. The method according to claims 1 to 4, wherein said decellularized extracellular matrix of an organ or of a portion of an organ is of a mammal, preferably from a sheep, pig, bovine, canine or human being.

6. The method according to claims 5, wherein said decellularized extracellular matrix of an organ or of a portion of an organ is selected from the group consisting of heart, lung, liver and kidney, and is preferably the liver.

7. The method according to claim 5 wherein the method comprises cannulating said organ at the suprahepatic vena cava (SHVC), the hepatic artery (HA), and the portal vein (PV).

8. The method according to any one of claims 1 to 6, wherein the cells are endothelial cells selected from the list consisting of umbilical vein endothelial cells (UVECs) and outgrowth endothelial cells (OECs), and stromal cells selected from the consisting of mesenchymal stem cells (MSCs) and smooth muscle cells (SMCs).

9. The method according to claim 7, wherein in step a) each cell type is divided for each of the following injection routes PV, SHVC and HA and injected or perfused in each of those injection routes at a concentration of between 1x10⁶ to 1x10⁸ during a time interval of 1 to 3 days.

10. The method according to claim 8, wherein said administration is carried out by injecting per injection route, in one or more injections per day, the total or a portion of the total amount of cells during a time interval of 1 to 3 days, at decreasing pressures within the range of between 150 and 10 mmHg.

11. The method according to claim 9, wherein the cells are administered during a time interval of three days, wherein on day 1 about 33% of the total amount of UVEC cells, 33% of the total amount of OECs cells, 50% of the total amount of MSC cells and 30% of the total amount of SMC cells are administered at a pressure of about 100 mmHg, wherein in day 2 about 33% of the total amount of UVEC cells, 33% of the total amount of OECs cells, 30% of the total amount of MSCs cells and 40% of the total amount of SMC cells are administered at a pressure of about 50 mmHg, and wherein in day 3 the rest of the cells of the total amount of cells are administered at a pressure of about 25 mmHg.

12. The method, according to claim 10, wherein each day the amount of cells are administered in at least 2-3 injections, and wherein each day one or more extra recirculation injections at the seeding pressure with basal medium are carried out to ensure proper cell penetration.

13. The method, according to any one of claims 8 to 11, wherein the total amount administered per each cell type is divided for each of the following injection routes PV, SHVC and HA in the following proportions, about 30% of the total amount of UVEC and OECS are administered via PV, about 50% of the total amount of endothelial cells are administered via SHVC, about 20% of the total amount of endothelial cells are administered via HA, about 40% of the total amount of MSC are administered via PV, about 40% of the total amount of stromal cells are administered via SHVC, and about 20% of the total amount of stromal cells are administered via HA. Also, about 30% of the total amount of SMC are administered via PV, about 30% of the total amount of stromal cells is administered via SHVC, and about 40% of the total amount of stromal cells are administered via HA.

14. A functional vascular tree within a re-cellularized extracellular matrix of an organ or of a portion of an organ obtained according to any one of claims 1 to 12.

15. A functional vascular tree according to claim 13, wherein the re-cellularized extracellular matrix is from a liver organ.

16. A functional vascular tree according to any one of claims 13 or 14 for use in organ transplantation in a subject in need thereof.
